(19) **Europäisches Patentamt / European Patent Office / Office européen des brevets**

(11) **EP 3 594 962 A1**

(12) # EUROPEAN PATENT APPLICATION

(43) Date of publication:
**15.01.2020 Bulletin 2020/03**

(51) Int Cl.:
*G16H 50/30* (2018.01)          *A61B 5/053* (2006.01)
*A61B 5/00* (2006.01)          *G16H 50/20* (2018.01)

(21) Application number: **18182886.4**

(22) Date of filing: **11.07.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventors:
• **Ouwerkerk, Martin**
  **5656 AE Eindhoven (NL)**
• **Natoewal, Navin Hemchand**
  **5656 AE Eindhoven (NL)**

• **Nieuwhof, Marc**
  **5656 AE Eindhoven (NL)**
• **Gordijn, Sandor Hurbertus Florentius**
  **5656 AE Eindhoven (NL)**
• **Penning de vries, Hendricus Theodorus Gerardus**
  **Maria**
  **5656 AE Eindhoven (NL)**
• **Rajae-joordens, Rosemarie Jolanda Elise**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **DEVICE, SYSTEM AND METHOD FOR DETERMINING A STRESS LEVEL OF A USER**

(57)    The present invention relates to devices, systems and methods for determining a stress level of a user and for determining a histogram for use in determining a stress level of a user. Stress-correlated data, e.g. psychophysiological data, such as skin conductance data, and a histogram of past data of the user are evaluated for this purpose.

FIG.1

**EP 3 594 962 A1**

**Description**

FIELD OF THE INVENTION

The present invention relates to a device, system and method for determining a stress level of a user. The present invention relates further to a device, system and method for determining a histogram for use in determining a stress level of a user. The present invention relates further to a wearable device and to a computer program.

BACKGROUND OF THE INVENTION

Mental stress and emotional stress, often called psychological stress, can compromise health when they are not adequately dealt with. Short periods of psychological stress can cause sleep disturbance, fatigue, headaches, and mood changes. Accumulated psychological stress can cause anxiety, depression, chronic fatigue, digestive problems, autoimmune disease, and cardiovascular disease. Psychological stress, further referred to as stress, and its related comorbid diseases are responsible for a large proportion of disability worldwide. This finding clearly underlines the need for pro-active reduction of stress by adequate coping to prevent stress to exert its harmful short and long-term effects on the body.

Stress is characterized by a series of bodily responses, including an increased heart rate, increased sweating (at multiple locations of the body) resulting in a rise in skin conductance, and the release of the stress hormone cortisol with a delay of 20-30 min. These bodily responses facilitate an adequate response towards an actual or perceived stressor, defined as an activity, event, or other stimulus that causes stress. Too high cortisol levels, however, are counter-productive, since they were associated with poorer memory performance and affect decision-making due to increased risk taking behavior.

Although the term stress for most people has a negative connotation, "stress" can generally be defined as "the non-specific response of the body to any demand for change". This implicates that the stress-induced bodily responses, including the cortisol release, are not limited to negative stress. Positive feelings, such as falling in love, sexual arousal, successful hunting in subsistence populations, and playing a table game, irrespective of whether the player won or lost indeed trigger the release of cortisol. As a consequence, adverse cognitive effects will also occur when cortisol levels become too high due to positive stress. Hence, stress may not only be mental or emotional stress, but may also be caused by other stressors, such as eating spicy food, stepping into a hot room, and vigorous activity.

These adverse effects make it important to keep a person's stress level within healthy boundaries. Accurately measuring a person's stress level is hampered by a lack of a yardstick. Often psychologists use validated questionnaires to obtain a person's perceived stress level. A more objective measure for stress is the serum or salivary cortisol level. The 20 to 30 minute delay makes real-time monitoring of stress using the cortisol level impossible.

US 2017/0071551 A1 discloses method for determining a stress level of a user based on a sympathovagal balance (SVB) value calculated based on a set of measurement data may include determining a heart-rate variability (HRV) characteristic as a ratio involving a number of autonomic nervous system (ANS) activity markers within a first portion of the set of measurement data and the number of ANS activity markers within a second portion of the set of measurement data, and then determining the stress level of the user based on the HRV characteristic. The first and second portions of the set of measurement data may be selected based on a user-specific baseline SVB value that divides a histogram representation of the set of measurement data into the first and second portions. Further, real-time monitoring is hampered with this approach because a data set needs to be recorded, which takes at least one minute of time.

SUMMARY OF THE INVENTION

It is an object of the present invention to provide improved devices, systems and methods as well as a wearable device that enable determining a stress level of a user in real-time. It is a further object to enable the determination of the user's stress level in an easy manner and with sufficient reliability.

In a first aspect of the present invention a device for determining a stress level of a user is presented comprising an interface configured to obtain a stress-related signal trace;
a processing unit configured to process the obtained stress-related signal trace by

- deriving a current stress-related signal value from the obtained stress-related signal trace, the current stress-related signal value representing a current value of a stress-correlated parameter of the obtained stress-related signal trace in a first time period,
- deriving a current stress-related signal value from the obtained stress-related signal trace, the current stress-related signal value representing a current value of a stress-correlated parameter of the obtained stress-related signal trace in a first time period,

- obtaining two or more stored percentile stress-related signal values, a stored percentile stress-related signal value representing the value of the stress-related parameter at one of two or more percentile levels of a stress-related signal histogram, the stress-related signal histogram representing a distribution of stress-related signal values derived in the past from a stress-related signal trace for a plurality of first time periods, wherein the two or more percentile levels in the stress-related signal histogram divide the stress-related signal histogram into three or more histogram sections, each histogram section covering a different range of the number of stress-related signal values and being assigned to a different stress level,
- comparing the current stress-related signal value to the obtained percentile stress-related signal values to determine the corresponding histogram section for said current stress-related signal value, and
- determining the current stress level of the user by determining the stress level assigned to the determined histogram section.

In a further aspect of the present invention a device for determining a histogram for use in determining a stress level of a user, in particular for use by a device for determining a stress level of a user as disclosed herein, said device comprising:

an interface configured to obtain a stress-related signal trace;
a processing unit configured to process the obtained stress-related signal trace by

- deriving a plurality of stress-related signal values from the obtained stress-related signal trace for a plurality first time periods over a second time period, a stress-related signal value representing a value of a stress-correlated parameter of the obtained stress-related signal trace in a first time period,
- forming a stress-related signal histogram from the derived stress-related signal values for the plurality of first time periods over the second time period, the stress-related signal histogram representing a distribution of stress-related signal values derived from the stress-related signal trace for a plurality of first time periods,
- determining two or more percentile levels in the stress-related signal histogram dividing the histogram into three or more histogram sections, each histogram section covering a different range of the number of stress-related signal values,
- determining percentile stress-related signal values, each representing the stress-related signal at one of the two or more percentile levels of the stress-related signal histogram, and
- storing the percentile stress-related signal values determined for the two or more percentile levels of the stress-related signal histogram.

In a further aspect of the present invention a system for determining a stress level of a user is presented comprising

- a sensor for acquiring a stress-related signal from a user; and
- a device as disclosed herein.

In a further aspect of the present invention a wearable device wearable by a user is presented, the wearable device comprising the system as disclosed herein.

In yet further aspects of the present invention, there are provided corresponding methods, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed methods, system, wearable device, computer program and medium have similar and/or identical preferred embodiments as the claimed devices, in particular as defined in the dependent claims and as disclosed herein.

The present invention provides a normalization and ranking method based on historic data of stress-correlated data, e.g. psychophysiological data, such as skin conductance data, heart rate variability data or breathing pattern data. To obtain a real-time or close to real-time assessment of the stress level measurement data related to the fast stress response, e.g. a psychophysiological stress response, are measured and evaluated. This fast response may be measured with an appropriate sensor, e.g. a psychophysiological sensor, such as a skin conductance sensor, which delivers a stress-related signal trace, which is evaluated according to the present invention. A stress-related signal in this context means a signal that is related in some way to and influenced by stress to which the user is exposed and from which information about stress of the user can be derived.

The proposed devices, systems and methods allow measuring and determining a person's stress level in real time. A person's stress responses are assessed using an appropriate modality, such as skin conductance. For some time (e.g. 6 to 10 hours of the day in an exemplary implementation), the cumulative stress responses per short time period (e.g. per minute in an exemplary implementation) are collected in a histogram. These histograms are collected and stored for a certain time period (e.g. the past 3 weeks in an exemplary implementation). The actual stress response (i.e. a current value for the sum of rising edges (SRE), which may be the result of some data processing of values taken from the actual stress-related signal trace) is compared with the historic data to obtain the stress level of a person. If for example the full stress spectrum comprises five stress levels, the stress level of a person is ranked in the highest level when the actual level exceeds the 80% percentile of the historic data represented by the histogram. The proposed ranking hence enables a real-time assessment of a person's stress level using a given histogram.

According to one aspect of the present invention the stress level is determined in real-time based on actual measurements. According to another aspect of the present invention a histogram is determined and stored, so that it can be used to determine a stress level later from an actual measurement of a stress-related signal. The proposed devices preferably comprise an interface for obtaining (i.e. retrieving or receiving) measurement data, in particular a stress-related signal trace, either directly from a sensor or a memory storing the measurement data.

In an embodiment of the device for determining a stress level of a user the processing unit is configured to obtain two or more stored percentile stress-related signal values obtained from stress-related signal values of a stress-related signal trace acquired on the same day of the week as the stress-related signal trace from which the current stress-related signal value is derived, and compare the current stress-related signal value to the obtained percentile stress-related signal values corresponding to the same day of the week. This improves the accuracy of the determination of the stress level.

In an embodiment of the device for determining a stress level of a user the processing unit is configured to obtain two or more stored percentile stress-related signal values obtained from stress-related signal values of stress-related signal traces acquired on two or more same days of the week as the stress-related signal trace from which the current stress-related signal value is derived, determine weighted average percentile stress-related signal values from the obtained percentile stress-related signal values, and compare the current stress-related signal value to the weighted average percentile stress-related signal values. Hereby, as one option the obtained percentile stress-related signal values may be equally weighted or by predetermined weights. The weighting makes the determination of the stress more accurate.

In an embodiment of the device for determining a histogram for use in determining a stress level of a user the processing unit is configured to determine and store percentile stress-related signal values for a plurality of second time periods. Preferably, the second time period is a time period in the range of one hour to one week, in particular in the range of 6 to 24 hours, preferably 6 to 10 hours. The histogram may thus comprise a larger collection of percentile stress-related signal values, which may partly or completely be used to determine the current stress level. A preferred period for obtaining data for the histogram may e.g. be 6 to 12 hours during the day.

The processing unit may further be configured to store, for a third time period, percentile stress-related signal values determined for a plurality of second time periods. Preferably, the third time period is a time period in the range of one day to one year, in particular in the range of one week to three months, preferably two or three weeks. Hence, the stress-related signal histogram data set may e.g. comprises a set of percentile stress-related signal values per day (each forming one stress-related signal histogram) for each of 21 or 28 days reflecting the user's stress states over the last 21 or 28 days, i.e. an stress-related signal histogram data set may comprise multiple stress-related signal histograms, e.g. one per day. These data can then be use to judge the user's current stress level based on actual measurements. Generally, storing histograms obtained for more than three months is less advantageous since seasonal influences start having an effect on the skin conductance level as well.

In another embodiment the processing unit is configured to assign different stress levels to the different histogram sections of the stress-related histogram, and store the assigned stress levels together with the percentile stress-related signal values. This allows a simple and real-time determination of the user's stress level based on a current measurement of a stress-related signal value.

The above-mentioned first time period is preferably a time period in the range of 10 seconds to 10 minutes, in particular in the range of 30 seconds to two minutes, preferably one minute. Hence, for the above-described computations the stress-related signal trace may be divided into signal portions of e.g. one minute, which are then evaluated separately to determine the current stress-related signal value and, from the current stress-related signal value, the current stress level.

In a preferred embodiment the stress-related signal trace is a skin conductance measurement trace and the stress-related signal value represents the sum of heights of rising edges, SRE, or the number of peaks or the average skin conductance level of the skin conductance measurement trace in the first time period. In this context the sum of rising edges (called SRE herein; sometimes also called SREH) represents the sum of heights of rising edges per time period (e.g. per minute).

An exemplary lower limit for a valid rising edge may be one second. A rising edge is a portion of the skin conductance trace, which rises without interruption. The rising edge height is the difference between the value of the skin conductance at the top of the rising edge and the value of the skin conductance at the bottom of the rising edge. The rising edge duration is the difference between the timestamp of the skin conductance data point of the top of the rising edge and the timestamp of the skin conductance data point at the bottom of the rising edge.

The current SRE value, as one embodiment of the stress-related signal value, can be obtained by adding the heights of all rising edges with this time period. Another option is to sum up the heights of rising edges for a shorter time period, e.g. for only the last 6 or 10 seconds, and then to multiply this sum with a corresponding factor of 10 or 6, respectively, to obtain the current SRE value for one minute. This current SRE value (for one minute) can then be compared with the percentile stress-related signal values (in this case percentile SRE values) of one or more histograms available for a longer time period, e.g. for three weeks, which has been obtained from earlier measurements of SRE values per minute. Generally, instead of 6 or 10 seconds of measurement, other time periods in the range of 1 to 60 seconds and a corresponding multiplication factor may be used.

For deriving an SRE value from the obtained stress-related signal trace for a first time period various options exist. According to a preferred option the processing unit is configured to derive an SRE value by determining a first derivate of the obtained stress-related signal trace, detecting zero crossings in said first derivate, detecting a rising edge by detecting a negative-to-positive zero crossing followed by a positive-to-negative zero crossing, and obtaining the SRE value by i) summing the heights of detected rising edges in the first time period or ii) summing the heights of detected rising edges in a time period shorter than the first time period and multiplying the sum by a multiplication factor represented by the ratio of the first time period divided by the shorter time period.

Hereby, the processing unit may be configured to add a detected rising edge to the sum only if the duration of the rising edge exceeds a minimum fourth time period, which is preferably a time period in the range of 0.1 seconds to 10 seconds, in particular in the range of 0.3 seconds to 1.5 seconds, preferably one second. Hence, very short rising edges, which may be noise or caused by disturbances, may be ignored to improve the reliability of the stress level determination. In exemplary embodiments 0.6 seconds or one second may be used as fourth time period, which is a reasonable time period for characterizing a valid peak. As upper limiting time period of the rising edge duration a value between 1.5 and 2 seconds may be used, but with high blood pressure or for young children such rising edges can become very short. For neonates it is about 0.25 seconds.

A set of two or more percentile levels of a stress-related histogram may comprise a predetermined number of percentile levels. Preferably, they are selected to subdivide the SRE histogram into equally large histogram sections. For instance, the set of percentile levels may comprise a 33% and 66% percentile level (i.e. two levels to have three histogram sections) or a 25%, 50% and 75% percentile level (i.e. three levels to have four histogram sections) or a 20%, 40%, 60% and 80% percentile level (i.e. four levels to have five histogram sections) or a 16.6%, 33.3%, 50%, 66.6% and 83.3% percentile level (i.e. five levels to have six histogram sections). Other numbers, e.g. more levels or non-equally distributed levels, such as logarithmically or exponentially arranged percentile levels and/or histogram sections, are possible as well.

In another embodiment, the stress-related signal trace is a heart rate variability trace and the stress-related signal value represents the high frequency to low frequency ratio of the heart rate variability trace in the first time period or wherein the stress-related signal trace is a breathing pattern trace and the stress-related signal value represents the respiration rate in the first time period or the stress-related signal trace is an electromyogram representing the muscle tension in the first time period.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of a system and a device according to the present invention;
Fig. 2 shows an exemplary implementation of a system according to the present invention in the form of a wearable device;
Fig. 3A shows a diagram of a skin conductance trace;
Fig. 3B shows a diagram of the calculated stress level for the same time interval as shown in Fig. 3A;
Fig. 4 shows a flow chart of an embodiment of a method for determining the stress level of a user according to the present invention;
Fig. 5 shows a flow chart of an embodiment of a method for for determining a histogram for use in determining a stress level of a user according to the present invention;
Fig. 6 shows a flow chart of another embodiment of a method according to the present invention;
Fig. 7 shows diagrams of a skin conductance trace, the determined stress level and the SRE values using absolute rising edge height;

Fig. 8 shows a diagram of a histogram corresponding to the diagrams shown in Fig. 7;

Fig. 9 shows diagrams of a skin conductance trace, the determined stress level and the SRE values using normalized rising edge height; and

Fig. 10 shows a diagram of a histogram corresponding to the diagrams shown in Fig. 9.

DETAILED DESCRIPTION OF THE EMBODIMENTS

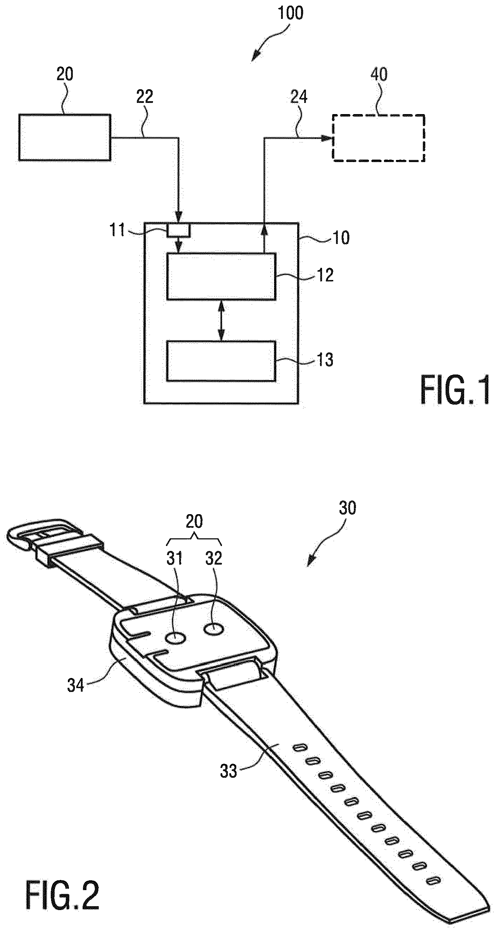

Fig. 1 shows a schematic diagram of exemplary embodiment of a system 100 according to the present invention. The system 100 comprises a sensor 20 for measuring a stress-related signal such as the skin conductance of the user. The stress-related signal measured by the sensor 20 over time forms a stress-related signal trace 22, which is generally indicative of one or more measured stimulus responses corresponding to a neural stress response. The system 100 further comprises a device 10 for determining a stress level of the user and/or for determining a histogram for use in determining the stress level. Preferably, the device 10 is used for both purposes.

The device 10 comprises an interface 11 for obtaining (i.e. receiving or retrieving from the sensor 20 or a (not shown) memory) the stress-related signal trace 22 and a processing unit 12 for processing the stress-related signal trace 22. The processing unit 12 can be any type of suitable processing unit or processor, such as for example a microprocessor/microcontroller, or embedded microcontroller but not limited thereto that is adapted accordingly. The interface 11 can be any kind of interface from obtaining data from the sensor 20 or a memory, e.g. a wireless or wired data interface or signal line. It will be understood that the sensor 20 and the device 10 can be part of the same device (e.g. wearable device or wristband) or can be implemented as or in separate devices. Details of the processing performed by the processing unit 12 will be explained below.

Optionally, as indicated by the dashed lines in Fig. 1, the system 100 can comprise an output unit 40 for outputting or rendering the determined stress level 24 to a user. For instance, an LED or buzzer, which can discreetly signal a stress level increase, may be used as output unit 40. The stress level may e.g. be communicated with a programmable LED signaling blue for the lowest level, then green, yellow, orange and red for the highest stress levels, in case of using five stress levels. It will be understood that the output unit 40 and the device 10 can be part of the same device (e.g. wearable device or wristband) or can be implemented as or in separate devices. For example, the output unit 40 of the system 100 may be implemented by means of a smartphone or other information processing entity at the same or a remote location. Correspondingly, the processing unit 12 can also be implemented by means of a smartphone that is adapted to perform the afore-mentioned functionality for example by running a corresponding application or another suitable computing device running the corresponding software.

The system 100 may further comprise a memory 13 for storing at least a histogram or particular data characterizing a histogram. Further, stress level data determined for the user in the past may be stored. Details will be explained below. The memory 13 can be part of the device 10 or can be an external memory. The memory can be any suitable memory such as for example a memory register or RAM (random access memory). It will be understood that the memory 13 and the processing unit 12 can be part of the same device (e.g. wearable device or wristband) or can be implemented as or in separate devices.

Fig. 2 shows an embodiment of a wearable device 30 wearable by user. In this embodiment, the wearable device 30 is implemented in the form of a smart watch. The smart watch comprises a wristband 33 and a casing 34. The wristband 33 can loop around the wrist of the user. It will be understood that a wearable device could also be worn around another suitable part of the body such as the ankle foot or hand or may be adapted for attachment to other parts of the body, e.g. in the form of a patch.

The wearable device 30 can comprise the proposed system 100 for determining a stress level of a user. In this way a corresponding system 100 can be provided in an unobtrusive and wearable format. Alternatively, the wearable device 30 may only comprise the sensor 20, in this embodiment a skin conductance sensor 20. The device 10 of the system 100 may be located at the remote location or implemented in a remote device (e.g. a remote computer, smartphone or patient monitor).

In the following, the invention will be explained in more detail by reference to a particular embodiment using a skin conductance trace as stress-related signal trace and sum of rising edges (SRE) values (actually representing the sum of the heights of the rising edges within a time period) as stress-related signal values.

The sensor 20 may be a skin conductance sensor 20 may comprise a first and a second skin conductance electrode 31, 32 in combination with a skin conductance measuring unit (not shown). In the embodiment of Fig. 2, two skin conductance electrodes 31, 32 are integrated into the casing of the wearable device, however is also possible to integrate them for example into the wristband 33 so that they contact the underside of the wrist. The skin conductance electrodes 31, 32 can be arranged so as to contact the upper side of the wrist when the user wears the wearable device 30. An exemplary implementation of a wearable device comprising a skin conductance sensor is the Philips discreet tension indicator DTI-4.

The skin conductance sensor 20 is adapted to measure the skin conductance of the user 2 between the skin conductance electrodes 31, 32. For this purpose, the skin conductance measuring sensor may comprise a voltage generator for applying a voltage between the at least two skin conductance electrodes, a sensing unit for sensing a current between the at least two electrodes, and/or a calculating unit for calculating the skin conductance based on the sensed current. The measured skin conductance over time forms, in this embodiment, the stress-related signal trace (or data). The stress-related signal trace (or data) may for example be stored in a memory of the wearable device 30, or may be transmitted (wirelessly or through a wire or signal line) to an external unit.

The skin conductance measuring sensor 20 and/or the device 10 (as shown in Fig. 1) may be integrated into the casing 34 of the wearable device 30. The wearable device 30 can further comprise a transmitter for transmitting data over a wireless or wired communication link, such as the measurement data, the determined SRE values, the histogram data and/or the determined stress level 16 of the user. However, it will be understood that the device 10 or processing unit 12 can also be implemented as or in separate parts or devices and that the wearable device 30 then transmits the stress-related data to the separate part or device via the transmitter.

Advantageously, the system 100 may also comprise an output unit 40 for outputting the stress level of the user. The output unit 40 may be a separate device or may be integrated into, for example, the wearable device 30 comprising the sensor 20 in form of a smart watch. Furthermore, an external output unit 40, for example a smartphone, tablet or PC running a corresponding application, may be used and coupled to the device 10 or wearable device 30.

In the following, details of the proposed approach will be explained using skin conductance data as exemplary stress-related measurement data, in this example psychophysiological measurement data. To derive the intensity of experienced emotions (in particular stress) of a person as measured from skin conductance data a classification method is used. According to this classification, the determined stress level (sometimes also called emotional intensity) may have an integer value in the range 1 to 5 in this exemplary embodiment. Other values and ranges are generally possible.

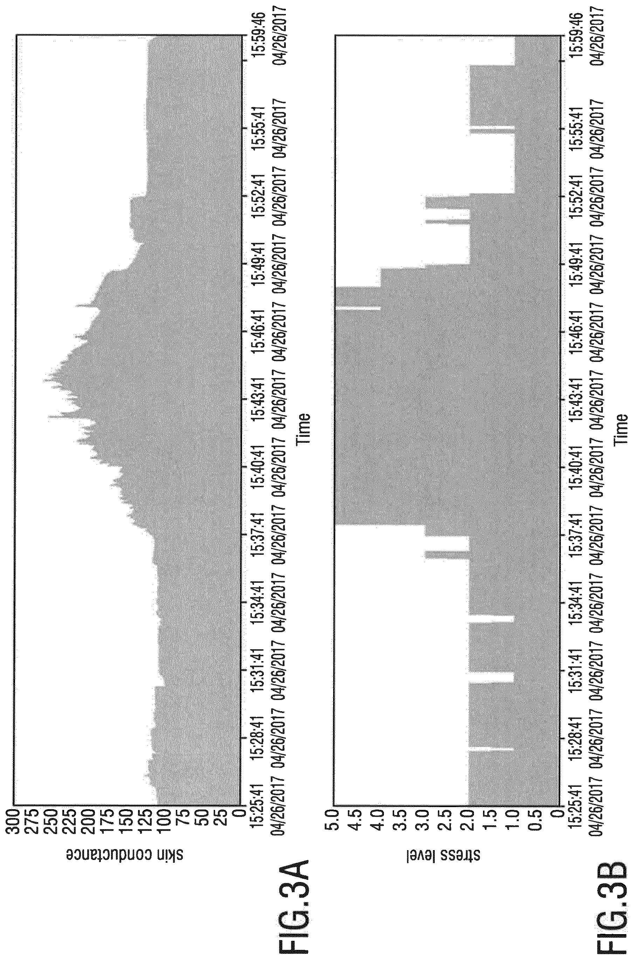

Fig. 3 shows a skin conductance trace (Fig. 3A) and the calculated stress level (Fig. 3B) for the same time interval. It can be seen that the responsiveness is fast, but the stress level value seems to saturate at the highest value.

In an embodiment a personal bandwidth is used for the stress level. If for a person the personal range of stress level were known, only truly intense emotions would yield a maximal stress level value. Hence, in this embodiment a person's personal range of stress level is derived when a certain amount of skin conductance data, e.g. skin conductance data of a day or more, becomes available.

In the following more details of embodiments of the present invention will be described, in particular of the determination and use of a histogram for determining a user's stress level. According to an aspect of the present invention a histogram of past data is used, i.e. event detection is based on comparing actual value with a histogram of past data. Further, for the calculation of the stress level steps are applied, according to which SRE values, an SRE value representing the sum of the heights of rising edges per time period (e.g. per minute), are used instead of the skin conductance level.

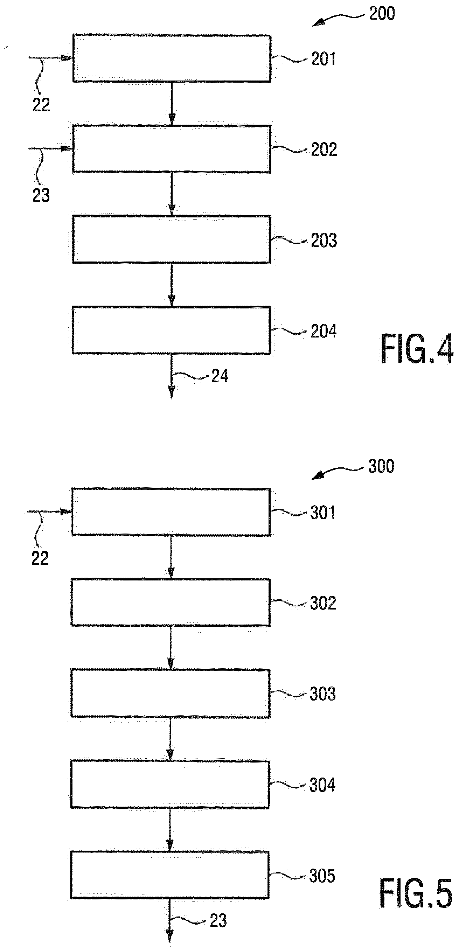

Fig. 4 shows a flow chart of an embodiment of a method 200 for determining the stress level of a user according to the present invention. The processing unit 12 of the device 10 shown in Fig. 1 may carry out this method 200.

In a first step 201 a current sum of rising edges (SRE) value is derived from an obtained stress-related signal trace 22. The current SRE value represents the current SRE in the obtained stress-related signal trace in a first time period, e.g. one minute. This current SRE value may be obtained by measuring the SRE in a shorter time interval, e.g. in a 6 to 10 seconds interval, in which the SRE value is calculated and then multiplied by a multiplication factor (e.g. 10 for a 6 seconds interval or 6 for a 10 seconds interval) to obtain the current SRE value per minute and to enable comparison with the histogram which is made up from per minute values.

In a second step 202 two or more stored percentile SRE values are obtained, in particular from an SRE histogram 23. A stored percentile SRE value represents the SRE at one of two or more percentile levels of an SRE histogram 23. The SRE histogram may e.g. be stored in a memory (e.g. memory 13 shown in Fig. 1), from which it is obtained.

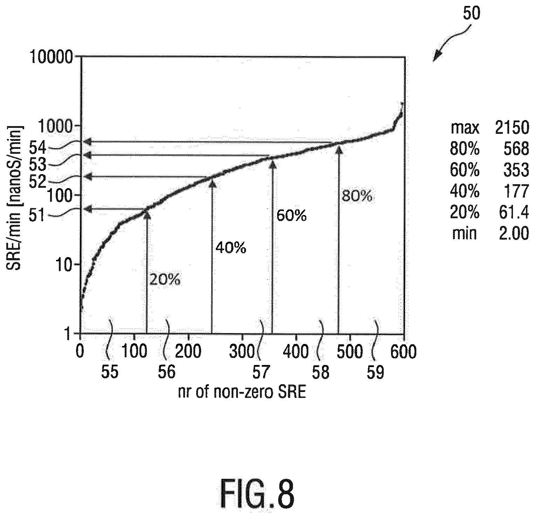

An SRE histogram represents a distribution of SRE values derived in the past from a stress-related signal trace for a plurality of first time periods. An exemplary SRE histogram 50 is shown in Fig. 8. On the x-axis the number of non-zero SRE values for a second time period (here 10 hours, i.e. in total up to 600 SRE values with one SRE value per minute; generally, it will be less than 600 SRE values since zero values are omitted in this embodiment) are shown and on the y-axis the SRE values per first time period (here one minute) for the plurality (here 600) of first time periods covered by the second time period (here 10 hours) are shown. The two or more (here four) percentile SRE values 51, 52, 53, 54 in the SRE histogram 50 divide the SRE histogram into three or more (here five) histogram sections 55, 56, 57, 58, 59. In the SRE histogram 50 shown in Fig. 8 four percentiles 20%, 40%, 60% and 80% are used, for which the corresponding percentile levels 51, 52, 53, 54 are determined. Hence, each histogram section 55, 56, 57, 58, 59 covers a different range of the number of SRE values and is assigned to a different stress level. For instance, histogram section 55 is assigned to stress level 1 (low stress) and histogram section 59 is assigned to stress level 5 (high stress). According to one option the complete SRE histogram may be stored in a memory (e.g. memory 13 shown in Fig. 1). It is generally, however, sufficient to store, as provided in another option, only the four percentile SRE values 51, 52, 53, 54, which allow distinguishing the five histogram sections and thus determining the current stress level if a current SRE value is

7

known.

Hence, in a subsequent step 203 the current SRE value is compared to the obtained percentile SRE values (i.e. the four percentile SRE values 51, 52, 53, 54 of the exemplary SRE histogram shown in Fig. 8) to determine the corresponding histogram section for said current SRE value. For instance, if the current SRE value is 100, it lies between the first percentile SRE value 51 and the second percentile SRE value 52 and, hence, in the second histogram section to which stress level 2 is assigned. In other words, if SRE value 100 is currently measured for a user, it can instantaneously be determined from the SRE histogram that the user has stress level 2. This is done in step 204, in which the current stress level 24 of the user is determined by determining the stress level assigned to the determined histogram section.

The SRE value can be calculated from absolute heights (in μS) or normalized (also called relative) heights (as percentage increase, unit less). In an implementation 100 levels may be used, and then the level would mean a stress percentage indicating if a person is stressed at a percentage between 0% (not stressed) and 100% (very stressed).

Various embodiments and modifications of this method exist. For instance, if a plurality of SRE histograms for multiple days is stored, to determine the current stress level an SRE histogram of the same day of the week as the current day may be chosen, or an average (or weighted average) of SRE histograms of a number of same days of the weeks may be formed and used. Preferably, only SRE histograms that have been obtained from the same user are used, while in an embodiment SRE histograms for a plurality of other persons with similar or identical personal features (e.g. age, weight, health status, gender, profession, hobbies, etc.) may be used. Such histograms can also serve to give meaningful data to a user on the first day of use. Histograms may be stored on an external server in the cloud or locally in a local network or on the user's device itself. Further, in an embodiment, stress (instead of the SRE) can also be derived from a skin conductance trace by the rising edge frequency per minute (peak frequency) or the average skin conductance level per minute.

Fig. 5 shows a flow chart of an embodiment of a method 300 for determining a histogram for use in determining a stress level of a user according to the present invention. This method 300 may be carried out by the processing unit 12 of the device 10 shown in Fig. 1 as well. Preferably, both methods 200 and 300 are carried out by the processing unit 12 of the device 10, which thus collects data for generating SRE histograms and determines current stress levels. The methods 200 and 300 may alternatively be carried out by separate devices.

In a first step 301 a plurality of SRE values are derived from an obtained stress-related signal trace 22 for a plurality first time periods over a second time period. In a second step 302 an SRE histogram is formed from the derived SRE values for the plurality of first time periods over the second time period. In a third step 303 two or more percentile levels are determined in the SRE histogram dividing the histogram into three or more histogram sections. In a fourth step 304 percentile SRE values are determined, each representing the SRE at one of the two or more percentile levels of the SRE histogram. In a fifth step 305 the percentile SRE values determined for the two or more percentile levels of the SRE histogram are stored. This SRE histogram, described by the stored percentile SRE values, may then be used as SRE histogram 23 in the method 200 illustrated in Fig. 4.

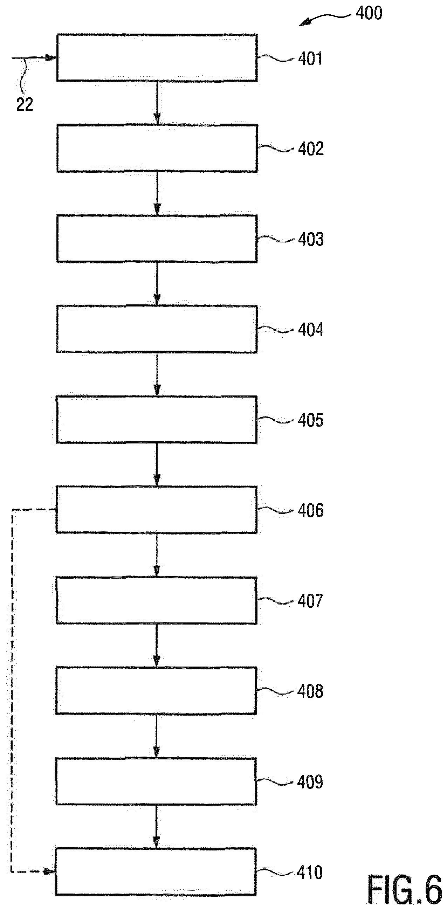

Fig. 6 shows a flow chart of another, more detailed embodiment of a method 400 according to the present invention.

In a first step 401 the skin conductance data are obtained, e.g. on the fly as currently measured by the sensor 20. The skin conductance data over time form a skin conductance trace 22.

In an optional step 402 high frequency noise may be filtered out from the skin conductance trace. For instance, 0.5 Hz can be used as cross-over.

In step 403 the sum of (normalized or absolute) rising edges (SRE) per first time period (e.g. per minute) is derived from the skin conductance trace 22 of the measured skin conductance data using the first derivative of the skin conductance.

In step 404 zero crossings of the first derivate of the skin conductance trace are determined. A negative to positive zero crossing of the first derivative of the time dependence of the skin conductance trace signals the onset of a rising edge. The next zero crossing (positive to negative) of the first derivative of the time dependence of the skin conductance trace signals the end of the rising edge.

In step 405, using a linear method, the skin conductance value at the onset is subtracted from the skin conductance value at the end of the rising edge to generate the rising edge height. Alternatively, using a logarithmic method, a rising edge of the skin conductance trace can be quantified by dividing the end value or the height with the onset value. In still another alternative using normalization, the skin conductance value at the onset may be extracted from the skin conductance value at the top and this is divided by the onset value.

In step 406 all rising edge heights in a first (longer) time period, e.g. in a minute, for which the criterion (called "duration criterion") is met that the duration of the rising edge exceeds a second (shorter) time period, e.g. one second (generally a duration in the range of 0.1 seconds to 10 seconds, in particular in the range of 0.5 seconds to 1.5 seconds), are summated to generate the sum of (valid) rising edge heights per first time period, e.g. per minute. It shall be noted, however, that a rising edge may continue into the next first time period, e.g. into the next minute. If this rising edge meets the duration criterion, its contribution until the end of the first time period, e.g. the end of the minute, is attributed to that first time period, e.g. to that minute, in step 406. The remainder of this rising edge is then attributed to the next minute. If this rising edge does not meet the duration criterion, it is not counted in the summation, but is considered to be caused by noise or motion artifacts.

In step 407, for a certain (e.g. preset) third time period (e.g. in the range of one day to one year, in particular in the range of one week to one month, preferably two or three weeks), the sums of rising edges per first time period are stored in a memory, e.g. put into a FIFO (first in - first out) buffer.

In an implementation a preferred setting is a period of a 10-hour day (i.e. the second time period is 10 hours) and one data point per minute (i.e. the first time period is one minute). A criterion for a minimal valid day (representing a "valid day criterion") may be set at a minimum of 6 hours in the time window 8 AM to 6 PM. This would give 360 to 600 data points for a valid day. Another exemplary time window may be 6 AM to 12 AM. The second time period does not need to be uninterrupted, but shall preferably be within the same day to average the values of the same day of the week if available. Another histogram comparison can be specified for the night: 6 hours between 12 PM and 6 AM to compare actual sleep data with historic data. Other options for forming a histogram may be a histogram for a full week, full month, full season or full year.

As soon as the valid day criterion is met a four-value down sampled version of the SRE histogram can be stored in non-volatile memory, e.g. the memory 13 in step 408. In this step, for an SRE histogram, the percentile SRE values are derived at the desired percentile levels (e.g. 20%, 40%, 60% and 80%), which are the four samples that characterize the SRE histogram (as explained above with reference to Fig. 8). These four percentile SRE values are then stored in the memory for this particular day in step 409.

Since the stress level shall be in the range 1 to 5 (or 0 to 4) in an embodiment of the classification, only four percentile SRE values (also called cross-over values herein) are stored. For example, the cross-over for 1 to 2 would be the SRE value per minute closest to the 20% percentile. In a histogram of 360 values this is the 60th value, and for 1440 values this is the 288th value. Accordingly, the 2 to 3 cross-over would be the 40% percentile, or the 120th/360th of 576/1440 values, etc. This is illustrated in Table 1 showing the SRE histogram based stress level cross-overs for a five-fold split, i.e. where the SRE histogram is split into five histogram sections. For 6 hours up to 360 histogram values can be obtained, for 10 hours up to 600 histogram values can be obtained, and for a full day up to 1440 histogram values can be obtained.

Table 1

| stress level | Low cross-over percentile | High cross-over percentile |
|---|---|---|
| 1 | 0 | 20 |
| 2 | 20 | 40 |
| 3 | 40 | 60 |
| 4 | 60 | 80 |
| 5 | 80 | 100 |

When these valid day data are stored the valid day count is increased by one. If the data collection continues after the valid day data storage moment the histogram continues to increase in size. Every two hours until midnight (0:00 AM) the valid day data can be recalculated using the then available histogram and stored into non-volatile memory. Ultimately, at 0:00 AM a final version can be calculated and stored.

An empty new histogram starts at 0:00 AM or when the device is switched on. Interruptions in data collection (not worn status) are ignored up to a certain duration, e.g. a duration of one hour. After that an empty new histogram starts when worn status is detected again.

Upon start of the device on a day where histogram data is available in non-volatile memory the current sum of rising edges per minute value can be ranked with respect of these values in step 410, which may immediately follow step 406. For example, an SRE value per minute in between the cross-over values 2-3 and 3-4 gets a stress level value of 3.

The daily cross-over values may be stored for each day of the week. Hence, there may be a valid day count for each day of the week. On a Monday the stored Monday values are then used if available. This allows working days and leisure days to be discriminated. Averaging daily cross-over values may be done as soon as they become available, i.e. when valid day count exceeds one. A maximum of multiple days, e.g. three weeks, of historic data may be stored.

In an embodiment the average value for a day is calculated in a weighted manner:

$$\text{weighted average } t = \tfrac{1}{2}\,(t\text{-}1) + 1/3\,(t\text{-}2) + 1/6\,(t\text{-}3)$$

where t is a specific day of the week, for example Tuesday. If more than 3 weeks are to be stored, the above-mentioned formula may be extended by more terms in the sum (one term per week). In Table 2 an example of the calculation is shown for a full data set, in particular a histogram for deriving a personal stress level range.

Table 2

| date | 20 | 40 | 60 | 80 | day of the week |
|---|---|---|---|---|---|
| 23-03-2017 | 3.3 | 6.5 | 13.2 | 39.4 | Thursday |
| 30-03-2017 | 7 | 25.1 | 72.5 | 213.1 | Thursday |
| 06-04-2017 | 6.9 | 18.3 | 48.7 | 103.7 | Thursday |
| 13-04-2017 | 7 | 19.7 | 40.7 | 100.5 | Thursday |
| weighted av. week 1-3 | 6.31 | 18.52 | 50.48 | 128.74 | Thursday |
| weighted av. week 2-4 | 6.96 | 21.93 | 59.25 | 157.84 | Thursday |
| 24-03-2017 | 3.2 | 5.5 | 13.5 | 29.7 | Friday |
| 31-03-2017 | 6 | 13.4 | 23.4 | 45.8 | Friday |
| 07-04-2017 | 2.5 | 4.5 | 7.5 | 16.2 | Friday |
| 14-04-2017 | 4.2 | 18.2 | 42.9 | 99.7 | Friday |
| weighted av. week 1-3 | 3.78 | 7.63 | 13.79 | 28.30 | Friday |
| weighted av. week 2-4 | 3.93 | 12.83 | 27.85 | 62.88 | Friday |
| 25-03-2017 | 20.3 | 43 | 70.45 | 184.26 | Saturday |
| 01-04-2017 | 8.2 | 23.4 | 48.1 | 116.3 | Saturday |
| 08-04-2017 | 6.8 | 17.3 | 36.9 | 76.71 | Saturday |
| 15-04-2017 | 2.3 | 3.6 | 6 | 9.8 | Saturday |
| weighted av. week 1-3 | 9.51 | 23.61 | 46.21 | 107.80 | Saturday |
| weighted av. week 2-4 | 4.78 | 11.46 | 23.31 | 49.83 | Saturday |
| 26-03-2017 | 9.1 | 21.2 | 48.05 | 85.55 | Sunday |
| 02-04-2017 | 12.7 | 33.5 | 63.25 | 117.164 | Sunday |
| 09-04-2017 | 2.2 | 3.1 | 4.2 | 5.9 | Sunday |
| 16-04-2017 | 21.1 | 53.1 | 135.98 | 239.208 | Sunday |
| weighted av. week 1-3 | 6.85 | 16.24 | 31.17 | 56.23 | Sunday |
| weighted av. week 2-4 | 13.40 | 33.17 | 79.93 | 141.10 | Sunday |
| 27-03-2017 | 5.1 | 11.11 | 33.5 | 103.3 | Monday |
| 03-04-2017 | 5.3 | 11.6 | 25.9 | 94.4 | Monday |
| 10-04-2017 | 14.3 | 48.5 | 123.68 | 323.81 | Monday |
| 17-04-2017 | 34.6 | 79.4 | 127.5 | 228.7 | Monday |
| weighted av. week 1-3 | 9.77 | 29.96 | 76.05 | 210.56 | Monday |
| weighted av. week 2-4 | 22.95 | 57.78 | 109.25 | 237.92 | Monday |
| 28-03-2017 | 6.5 | 13.8 | 35.4 | 87.6 | Tuesday |
| 04-04-2017 | 9.6 | 25.2 | 79.95 | 275.31 | Tuesday |
| 11-04-2017 | 5.9 | 14.2 | 26.5 | 86.9 | Tuesday |
| 18-04-2017 | 17.6 | 61.1 | 163.7 | 475.5 | Tuesday |
| weighted av. week 1-3 | 7.23 | 17.79 | 45.77 | 149.73 | Tuesday |

(continued)

| date | 20 | 40 | 60 | 80 | day of the week |
|------|-----|-----|--------|--------|-----------------|
| weighted av. week 2-4 | 12.37 | 39.48 | 104.00 | 312.58 | Tuesday |
| 29-03-2017 | 8.3 | 27.5 | 65 | 120.7 | Wednesday |
| 05-04-2017 | 2.1 | 3 | 4.6 | 8.9 | Wednesday |
| 12-04-2017 | 13.4 | 37.7 | 95.74 | 241.16 | Wednesday |
| weighted av. week 1-3 | 8.78 | 30.68 | 69.52 | 282.13 | Wednesday |

Using the proposed method, a personal range of skin conductance values is determined from which a personal stress level (stress level value), e.g. between 1 and 5, can be derived. Minor emotions will give low stress level and only strong enough emotions to cross the 80% percentile rank the highest value, thus preventing unwanted saturation.

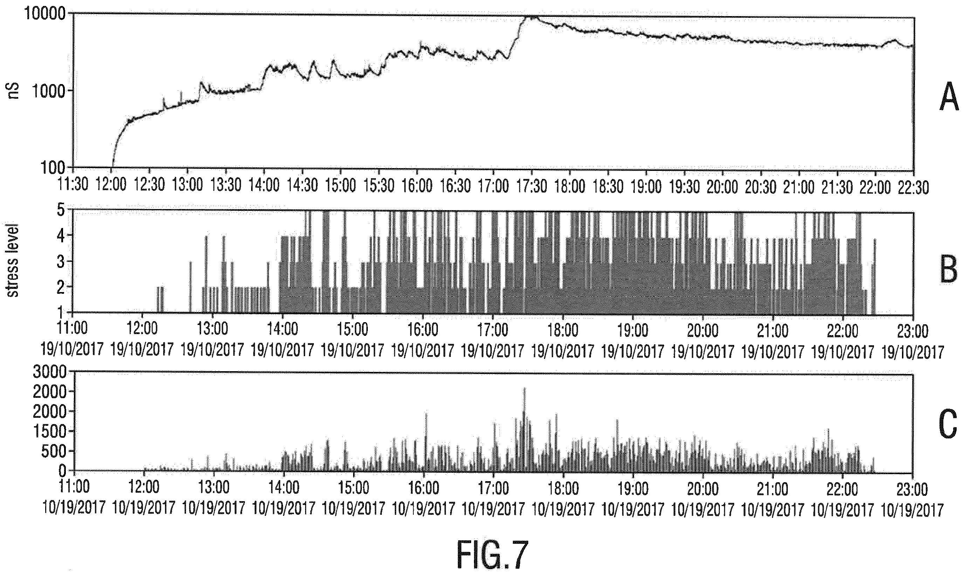

Fig. 7 shows diagrams of a skin conductance trace (A), the determined stress level (B) and the SRE values (C) using absolute rising edge height. Fig. 8 shows a diagram of a histogram 50 corresponding to the diagrams shown in Fig. 7. In this embodiment, per minute the cumulative increase of the skin conductance is calculated by adding up the absolute rising edge height. Rising edges (sometimes also called peaks) that are still rising at the end of a minute are split into two parts that are both taken into account. All positive sums are arranged in order of size. The 20%, 40%, 60% and 80% percentiles are the cross-overs for stress levels 2, 3, 4 and 5 respectively.

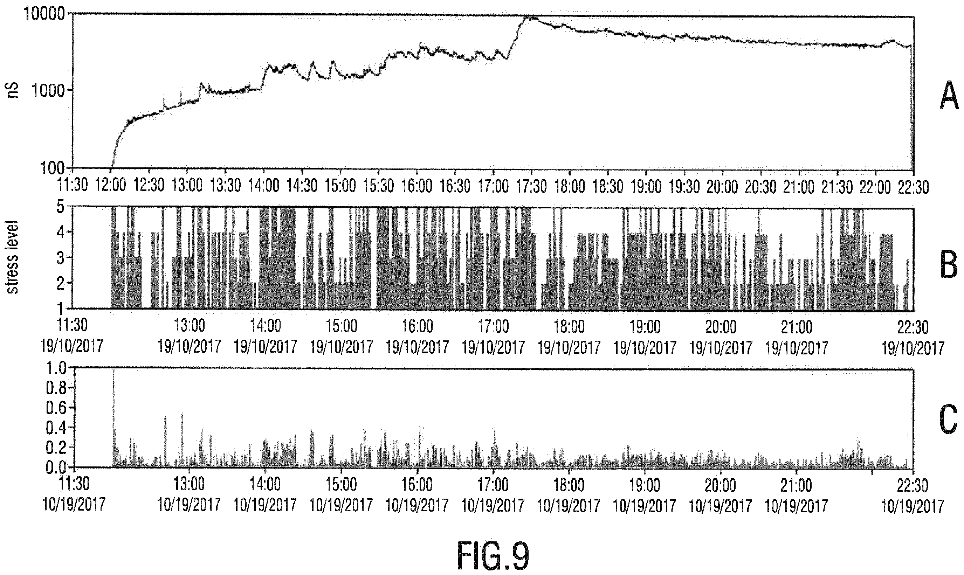

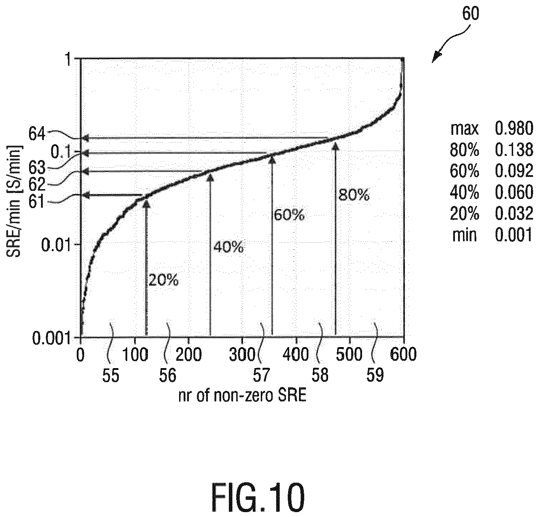

Fig. 9 shows diagrams of a skin conductance trace (A), the determined stress level (B) and the SRE values (C) using normalized rising edge height. Fig. 10 shows a diagram of a histogram 60 corresponding to the diagrams shown in Fig. 9. In this embodiment, per minute the cumulative increase of the skin conductance is calculated by adding up the normalized rising edge heights (=rising edge height / base level). The base level is the skin conductance value of the onset of a rising edge. Rising edges that are still rising at the end of a minute are split into two parts that are both taken into account. All positive sums are arranged in order of size. The 20%, 40%, 60% and 80% percentiles are the cross-overs for stress levels 2, 3, 4 and 5 respectively. As can be seen from a comparison of Figs. 7 and 9, the percentile SRE values 61-64 of the SRE histogram 60 are somewhat different from the percentile SRE values 51-54 of the SRE histogram 50.

In the above description of exemplary embodiments of the present invention skin conductance has been used as a measure of stress. Particularly the skin conductance peak frequency and the average level are commonly accepted measures to measure stress with skin conductance. The method can be applied for other known measures of stress, such as heart rate variability or breathing patterns with only minor or even no modifications.

For heart rate variability the frequency spectrum of the inverse of the inter beat intervals (i.e. the intervals between heart beats) in a certain time period, e.g. one minute, is used. Two frequency bands are used: a low frequency (LF) band, e.g. 0.04-0.15 Hz, and a high frequency (HF) band, e.g. 0.15-0.4 Hz. These frequency bands signify the rhythms of the variations in the inter beat intervals. The LF/HF ratio is reportedly linked to stress. Analogous to skin conductance the LF/HF ratio is calculated from each minute of the day trace of inter beat intervals. These values are arranged in ascending order, and the 20%, 40%, 60% and 80% values are stored and used the next day to rank the real/time LF/HF ratios in a 5 point stress level.

Another indicator for stress is muscle tension. Shoulder, lower back and some facial muscles get an increased tension under stress. Hence, an electromyogram (EMG) provides a measured parameter representing muscle tension, which may be evaluated according to the present invention.

For breathing patterns the average respiration frequency in each certain time period, e.g. one minute, may be taken, e.g. for each minute of the day. These values may be arranged in ascending order, and the 20%, 40%, 60% and 80% values may again be stored for use the next day to evaluate the actual respiration frequency and determine one of e.g. five stress levels from it.

All other embodiments and modifications described above mutually apply if heart rate variability or breathing pattern is used instead of skin conductance.

The present invention can also be used when no historic data are available. It can be started to build a histogram of sums of rising edges e.g. per minute upon startup until the valid day criterion is met and to adapt the cross over values as the histogram builds. Further, valid day or updated cross-over percentile values can be stored e.g. to a non-volatile memory with a time stamp, e.g. a day of week marker. Valid days may be stored for each day of day of the week. The number of valid days may be increased when a new valid day becomes available. When historic data is present the actual sum of rising edges value may be ranked to it to get the stress level. Average new valid day data may be obtained with stored data using a moving average algorithm. A factory reset means all stored values are erased. If the used histograms are uploaded, e.g. to a server in the cloud or the internet for the entire user population, first day users can opt to use an average histogram based on data of their peers.

The present invention is particularly useful for use in lifestyle coaching or the treatment of aggression or signaling of anger prior to aggressive behavior. Generally, it can be used in all applications requiring or preferably using the stress level of a person.

Embodiments of the device according to the present invention may also take the following forms:
Device as disclosed herein,
wherein the second time period is a time period in the range of one hour to one week, in particular in the range of 6 to 24 hours.

Device as disclosed herein,
wherein the third time period is a time period in the range of one day to one year, in particular in the range of one week to three months.

Device as disclosed herein,
wherein the first time period is a time period in the range of 10 seconds to 10 minutes, in particular in the range of 30 seconds to two minutes, preferably one minute.

Device as disclosed herein,
wherein the fourth time period is a time period in the range of 0.1 seconds to 10 seconds, in particular in the range of 0.5 seconds to 1.5 seconds, preferably one second.

Device as disclosed herein,
wherein the two or more percentile levels of an stress-related signal histogram comprise a 33% and 66% percentile level or a 25%, 50% and 75% percentile level or a 20%, 40%, 60% and 80% percentile level or a 16.6%, 33.3%, 50%, 66.6% and 83.3% percentile level.

Device as disclosed herein,
wherein the stress-related signal trace (22) is a heart rate variability trace and the stress-related signal value represents the high frequency to low frequency ratio of the heart rate variability trace in the first time period or wherein the stress-related signal trace (22) is a breathing pattern trace and the stress-related signal value represents the respiration rate in the first time period or the stress-related signal trace (22) is an electromyogram representing the muscle tension in the first time period.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device for determining a stress level of a user, said device comprising:

   an interface (11) configured to obtain a stress-related signal trace (22);
   a processing unit (12) configured to process the obtained stress-related signal trace by

   - deriving a current stress-related signal value from the obtained stress-related signal trace, the current stress-related signal value representing a current value of a stress-correlated parameter of the obtained stress-related signal trace in a first time period,
   - obtaining two or more stored percentile stress-related signal values, a stored percentile stress-related signal value representing the value of the stress-related parameter at one of two or more percentile levels of a stress-related signal histogram, the stress-related signal histogram representing a distribution of stress-related signal values derived in the past from a stress-related signal trace for a plurality of first time periods, wherein the two or more percentile levels in the stress-related signal histogram divide the stress-related signal histogram into three or more histogram sections, each histogram section covering a different range of the number of stress-related signal values and being assigned to a different stress level,

- comparing the current stress-related signal value to the obtained percentile stress-related signal values to determine the corresponding histogram section for said current stress-related signal value, and
- determining the current stress level of the user by determining the stress level assigned to the determined histogram section.

**2.** Device as claimed in claim 1,
wherein the processing unit (12) is configured to

- obtain two or more stored percentile stress-related signal values obtained from stress-related signal values of a stress-related signal trace acquired on the same day of the week as the stress-related signal trace from which the current stress-related signal value is derived, and

compare the current stress-related signal value to the obtained percentile stress-related signal values corresponding to the same day of the week.

**3.** Device as claimed in any one of the preceding claims,
wherein the processing unit (12) is configured to

- obtain two or more stored percentile stress-related signal values obtained from stress-related signal values of stress-related signal traces acquired on two or more same days of the week as the stress-related signal trace from which the current stress-related signal value is derived,
- determine weighted average percentile stress-related signal values from the obtained percentile stress-related signal values, and

compare the current stress-related signal value to the weighted average percentile SRE values.

**4.** Device for determining a histogram for use in determining a stress level of a user, in particular for use by a device as claimed in claim 1, said device comprising:

an interface (11) configured to obtain a stress-related signal trace (22);
a processing unit (12) configured to process the obtained stress-related signal trace by

- deriving a plurality of stress-related signal values from the obtained stress-related signal trace for a plurality first time periods over a second time period, a stress-related signal value representing a value of a stress-correlated parameter of the obtained stress-related signal trace in a first time period,
- forming a stress-related signal histogram from the derived stress-related signal values for the plurality of first time periods over the second time period, the stress-related signal histogram representing a distribution of stress-related signal values derived from the stress-related signal trace for a plurality of first time periods,
- determining two or more percentile levels in the stress-related signal histogram dividing the histogram into three or more histogram sections, each histogram section covering a different range of the number of stress-related signal values,
- determining percentile stress-related signal values, each representing the stress-related signal at one of the two or more percentile levels of the stress-related signal histogram, and
- storing the percentile stress-related signal values determined for the two or more percentile levels of the stress-related signal histogram.

**5.** Device as claimed in claim 4,
wherein the processing unit (12) is configured to determine and store percentile stress-related signal values for a plurality of second time periods.

**6.** Device as claimed in any one of claims 4 or 5,
wherein the processing unit (12) is configured to store, for a third time period, percentile stress-related signal values determined for a plurality of second time periods.

**7.** Device as claimed in any one of claims 4 to 6,
wherein the processing unit (12) is configured to

- assign different stress levels to the different histogram sections of the stress-related signal histogram, and

- store the assigned stress levels together with the percentile stress-related signal values.

8.  Device as claimed in any one of the preceding claims,
    wherein the stress-related signal trace (22) is a skin conductance measurement trace and the stress-related signal value represents the sum of heights of rising edges, SRE, or the number of peaks or the average skin conductance level of the skin conductance measurement trace in the first time period.

9.  Device as claimed in any one of the preceding claims,
    wherein the stress-related signal value is an SRE value,
    wherein the processing unit (12) is configured to derive an SRE value from the obtained stress-related signal trace for a first time period by

    - determining a first derivate of the obtained stress-related signal trace,
    - detecting zero crossings in said first derivate,
    - detecting a rising edge by detecting a negative-to-positive zero crossing followed by a positive-to-negative zero crossing, and
    - obtaining the SRE value by i) summing the heights of detected rising edges in the first time period or ii) summing the heights of detected rising edges in a time period shorter than the first time period and multiplying the sum by a multiplication factor represented by the ratio of the first time period divided by the shorter time period.

10. Device as claimed in claim 9,
    wherein the processing unit (12) is configured to add a detected rising edge to the sum only if the duration of the rising edge exceeds a minimum fourth time period.

11. System for determining a stress level of a user, said system comprising:

    - a sensor (20) for acquiring a stress-related signal (22) from a user; and
    - a device (10) as claimed in any one of the preceding claims.

12. Wearable device (30) wearable by a user, the wearable device comprising the system (100) as claimed in claim 11.

13. Method for determining a stress level of a user, said method comprising:

    - deriving a current stress-related signal value from the obtained stress-related signal trace, the current stress-related signal value representing a current value of a stress-correlated parameter of the obtained stress-related signal trace in a first time period,
    - obtaining two or more stored percentile stress-related signal values, a stored percentile stress-related signal value representing the value of the stress-related parameter at one of two or more percentile levels of a stress-related signal histogram, the stress-related signal histogram representing a distribution of stress-related signal values derived in the past from a stress-related signal trace for a plurality of first time periods, wherein the two or more percentile levels in the stress-related signal histogram divide the stress-related signal histogram into three or more histogram sections, each histogram section covering a different range of the number of stress-related signal values and being assigned to a different stress level,
    - comparing the current stress-related signal value to the obtained percentile stress-related signal values to determine the corresponding histogram section for said current stress-related signal value, and
    - determining the current stress level of the user by determining the stress level assigned to the determined histogram section.

14. Method for determining a histogram for use in determining a stress level of a user, in particular for use by a method as claimed in claim 18, said device comprising:

    - deriving a plurality of stress-related signal values from the obtained stress-related signal trace for a plurality first time periods over a second time period, a stress-related signal value representing a value of a stress-correlated parameter of the obtained stress-related signal trace in a first time period,
    - forming a stress-related signal histogram from the derived stress-related signal values for the plurality of first time periods over the second time period, the stress-related signal histogram representing a distribution of stress-related signal values derived from the stress-related signal trace for a plurality of first time periods,
    - determining two or more percentile levels in the stress-related signal histogram dividing the histogram into three or more histogram sections, each histogram section covering a different range of the number of stress-related signal values,
- determining percentile stress-related signal values, each representing the stress-related signal at one of the two or more percentile levels of the stress-related signal histogram, and
- storing the percentile stress-related signal values determined for the two or more percentile levels of the stress-related signal histogram.

15. Computer program comprising program code means for causing a computer to carry out the steps of the method as claimed in claim 13 or 14 when said computer program is carried out on a computer.

FIG.1

FIG.2

FIG.3A

FIG.3B

200

201

22

202

23

203

204

24

FIG.4

300

301

22

302

303

304

305

23

FIG.5

FIG.6

FIG.7

FIG.8

FIG.9

EP 3 594 962 A1

FIG.10

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y<br>A | WO 2017/178359 A1 (KONINKLIJKE PHILIPS NV [NL]) 19 October 2017 (2017-10-19)<br>* page 1, line 26 - page 2, line 9 *<br>* page 6, lines 10-26 *<br>* page 11, lines 9-12 *<br>* page 13, lines 21-31 *<br>----- | 4-12,14,15<br>1,2,13<br>3 | INV.<br>G16H50/30<br>A61B5/053<br>A61B5/00<br>G16H50/20 |
| Y<br><br>A | US 2011/306846 A1 (OSORIO IVAN [US]) 15 December 2011 (2011-12-15)<br>* paragraphs [0002], [0046], [0098] - [0100] *<br>----- | 1,2,13<br><br>3-12,14,15 | |
| X<br><br>Y<br>A | US 2017/000398 A1 (OUWERKERK MARTIN [NL] ET AL) 5 January 2017 (2017-01-05)<br>* paragraphs [0008] - [0009], [0022] *<br>----- | 4-12,14,15<br>1,2,13<br>3 | |

TECHNICAL FIELDS
SEARCHED (IPC)

G16H
A61B

The present search report has been drawn up for all claims

| Place of search | Date of completion of the search | Examiner |
|---|---|---|
| Munich | 3 December 2018 | Reinbold, Bernhard |

EPO FORM 1503 03.82 (P04C01)

**EP 3 594 962 A1**

## ANNEX TO THE EUROPEAN SEARCH REPORT
## ON EUROPEAN PATENT APPLICATION NO.

EP 18 18 2886

This annex lists the patent family members relating to the patent documents cited in the above-mentioned European search report.
The members are as contained in the European Patent Office EDP file on
The European Patent Office is in no way liable for these particulars which are merely given for the purpose of information.

03-12-2018

| Patent document cited in search report | | | Publication date | Patent family member(s) | | | Publication date |
|---|---|---|---|---|---|---|---|
| WO 2017178359 | A1 | | 19-10-2017 | NONE | | | |
| US 2011306846 | A1 | | 15-12-2011 | US | 2011306846 | A1 | 15-12-2011 |
| | | | | US | 2015119658 | A1 | 30-04-2015 |
| US 2017000398 | A1 | | 05-01-2017 | CA | 2932284 | A1 | 11-06-2015 |
| | | | | CN | 105792737 | A | 20-07-2016 |
| | | | | EP | 3076868 | A2 | 12-10-2016 |
| | | | | JP | 6133512 | B2 | 24-05-2017 |
| | | | | JP | 2017501777 | A | 19-01-2017 |
| | | | | US | 2017000398 | A1 | 05-01-2017 |
| | | | | WO | 2015082231 | A2 | 11-06-2015 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170071551 A1